(19) Europäisches Patentamt
European Patent Office
Office européen des brevets

(11) **EP 4 246 142 A1**

(12) **EUROPEAN PATENT APPLICATION**

(43) Date of publication:
**20.09.2023 Bulletin 2023/38**

(21) Application number: **22161997.6**

(22) Date of filing: **14.03.2022**

(51) International Patent Classification (IPC):
*G01N 33/50* (2006.01)

(52) Cooperative Patent Classification (CPC):
**G01N 33/502**

| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN** | (71) Applicant: **GSK Consumer Healthcare SARL**<br>**1197 Prangins (CH)**<br><br>(72) Inventor: **The designation of the inventor has not yet been filed**<br><br>(74) Representative: **Haleon Patent Department**<br>**5 The Heights, Wellington Way**<br>**Weybridge, Surrey KT13 0NY (GB)** |

(54) **IN VITRO METHODS**

(57) The present invention relates to improved methods for characterising diffusion of compounds through the skin using open flow micro-perfusion (OFM) in conjunction with Diffusion Cell apparatus, particularly static Franz Diffusion cell (FDC). The methods are in vitro methods using ex vivo skin explants.

**EP 4 246 142 A1**

**Description**

**FIELD OF THE INVENTION**

The present invention relates to improved methods for characterising diffusion of compounds, e.g. of active pharmaceutical ingredients (APIs) through the skin using open flow micro-perfusion (OFM) in conjunction with Diffusion Cell apparatus, particularly static Franz Diffusion cell (FDC). The methods are in vitro methods using skin explants.

**BACKGROUND TO THE INVENTION**

The current medical product development path is becoming increasingly challenging, inefficient, and costly. In the opinion of the US Food and Drug Administration (FDA), *"Not enough applied scientific work has been done to create new tools to get fundamentally better answers about how the safety and effectiveness of new products can be demonstrated, in faster time frames, with more certainty, and at lower costs. In many cases, developers have no choice but to use the tools and concepts of the last century to assess this century's candidates. As a result, the vast majority of investigational products that enter clinical trials fail."* (USFDA; 2004).
This statement is particularly relevant for the development of efficacious topical products, especially those whose targeted site of action is situated underneath the skin, e.g., in the soft tissues or articulations.
The selection of clinical candidates relies largely on their ability to deliver the active pharmaceutical ingredient (API) across the skin barrier as evaluated using the Franz diffusion cell (FDC). Despite its respectable age (Franz 1975), it is still the most broadly applied workhorse in the process of topical drug development. Nonetheless, FDC has the limitation that whilst this methodology can provide reliable data related to passive diffusion of an API across the skin barrier (the stratum corneum or SC), it reveals little about the excipient-dependent API biodistribution further downstream, for example, in the deeper dermis, because the API will not easily be released from the lower skin layers and thus not be detectable in the receptor fluid.
Physiological based pharmacokinetic modeling and simulation (PBPK) is a computer modeling approach that incorporates blood flow and tissue composition of organs to define the pharmacokinetics (PK) of drugs. The use of PBPK is favored by FDA as an approach allowing reduction or elimination of costly and lengthy clinical trials. However, like FDC these methods also have limitations. For example, the current models for topical absorption comprise biological processes which are validated only by inference using plasma pharmacokinetics and only in rare instances, tissue sampling. Using model-generated predictions of local and systemic API bioavailability to compare complex pharmaceutical formulations having different compositions and/or physical and structural properties, is still a daunting task. Quantitative structure-activity relationship (QSAR) based estimations of the diffusion coefficient (D) and solute partition coefficient (K) for API transdermal diffusion in the presence of different inactive ingredients are still not sufficiently precise and often not even feasible in view of the synergistic effects ensuing in many excipient combinations.
There is thus a need for improved methods for characterising API delivery from products for topical administration to different layers of the skin.

**SUMMARY OF THE INVENTION**

The present inventors have discovered that open flow micro-perfusion (OFM) can be applied in conjunction with Diffusion Cell apparatus, particularly the Franz Diffusion cell (FDC), to provide improved in vitro methods for evaluating diffusion of compounds, such as APIs, into the skin. By combining these two methods (FDC and OFM) for the first time, a new, convenient method for evaluating the influence of excipients on the API passive diffusion beyond the SC barrier has been obtained - a possibility not readily available using either FDC or OFM alone.
Thus, in a First Aspect there is provided an in vitro method to evaluate diffusion of a compound into the skin, the method comprising:

(i) applying the compound to a surface of a first skin explant, determining the concentration of the compound within the first skin explant using a Diffusion Cell (DC); and
(ii) applying the compound to a surface of a second skin explant, determining the concentration of the compound within the second skin explant using open flow micro-perfusion (OFM).

The compound may be an active pharmaceutical ingredient (API). Particularly, the compound is part of a composition comprising the compound and at least one pharmaceutically acceptable carrier. More particularly, the compound is part of a composition formulated for topical application. In some embodiments, the compound is diclofenac or salt thereof. Particularly, the compound is diclofenac sodium or diclofenac diethylammonium.
According to another aspect of the invention, an in vitro method for determining a skin permeation profile for a topical pharmaceutical composition comprising an active ingredient and at least one pharmaceutically acceptable carrier comprises the following steps:

(i) obtaining time-resolved concentration data for the active ingredient within the upper dermis of a first skin explant, wherein the concentration data is obtained using a diffusion cell;
(ii) obtaining time-resolved concentration data for the active ingredient within the lower dermis and/or subcutis of a second skin explant, wherein the concentration data is obtained using open flow micro-perfusion;
(iii) generating a time-resolved profile of concentration data for the active ingredient as a function of time within the upper dermis, lower dermis and/or subcutis.

Preferably, the open flow micro-perfusion utilises a perfusate comprising 1% human serum albumin.

Preferably, the Diffusion Cell apparatus is a Franz Diffusion Cell (FDC).

The first and second skin explants may be different parts of the same skin explant or two different skin explants. Skin explants used in the methods of the invention for DC, particularly FDC, comprise an epidermal layer having an outer stratum corneum and an upper dermal layer. Skin explants used in the methods of the invention for OFM comprise an epidermal layer having an outer stratum corneum, an upper dermal layer, a lower dermal layer and a subcutaneous layer.

Steps (i) and (ii) may be performed in any order, for example, sequentially (i.e. i then ii or ii then i) or in parallel (i.e. at the same time).

Preferably, the surface of the skin explant(s) to which the compound is applied is the stratum corneum. Thus, the method may comprise applying the compound to the stratum corneum of a first skin explant and/or applying the compound to the stratum corneum of a second skin explant.

Particularly, the concentration of the compound within the skin explant(s) is determined at sites within the skin explant(s) distal to the stratum corneum, for example, within the epidermal layer, upper dermal layer, lower dermal layer and/or subcutaneous layer.

In certain embodiments, the method comprises determining the concentration of the compound within the upper dermal layer of the first skin explant using a Diffusion Cell (DC). In certain embodiments, the method comprises determining the concentration of the compound within the upper dermal layer of the first skin explant using a Franz Diffusion Cell (FDC). The term "upper dermal layer" is used herein to designate an upper portion of the dermis, for example within a range of 0.2mm to 0.4mm from the stratum corneum surface.

The method may comprise determining the concentration of the compound within the dermal layer, preferably a lower dermal layer, of the second skin explant using open flow micro-perfusion (OFM). The term "lower dermal layer" is used herein to designate a lower portion of the dermis, between 0.5mm and 1.5mm, preferable between 0.8mm and 1.2mm, preferably around 1mm from the upper surface of the stratum corneum. The method may comprise determining the concentration of the compound within the subcutaneous layer of the second skin explant using open flow micro-perfusion (OFM). The measure within the subcutaneous layer can for example been taken at between 2.5 mm to 3.5 mm from the upper surface of the stratum corneum.

In preferred embodiments, the method comprises determining the concentration of the compound within the lower dermal layer and subcutaneous layer of the second skin explant using open flow micro-perfusion (OFM).

Particularly, the concentration of the compound within the skin explant is determined more than once, for example, 2, 3, 4, 5, 6, 7, 8, 9, 10, 11, 12, 13, 14 or more times.

Particularly, the concentration of the compound within the skin explant is determined more than once at a plurality of different time-points. The concentration can for example be determined once per hour, or every 2 hours, for a total time period of at least 12 hours or preferably at least 24 hours.

In some embodiments there is provided an in vitro method to evaluate diffusion of a compound into the skin, the method comprising:

(i) applying the compound to a surface of a first skin explant, determining the concentration of the compound within the first skin explant at a plurality of different time-points using a Diffusion Cell (DC); and
(ii) applying the compound to a surface of a second skin explant, determining the concentration of the compound within the second skin explant at a plurality of different time-points using open flow micro-perfusion (OFM).

More particularly, the steps of determining the concentration of the compound within the skin explant(s) are repeated at different points in time over a period of time starting from the time at which the compound was first applied to the surface of the skin explant(s), for example, over a period of 24 hours. In some embodiments the step of determining the concentration of the compound is repeated hourly over a time period of 24 hours. Preferably, a sequence of measurements is obtained at a series of time intervals and used to generate a time-resolved profile of the concentrations of the compound. The time-resolved profile shows the concentration of the compound as a function of time at a number of locations within the skin explant(s). Particularly, data generated by OFM and FDC are combined to provide a time-resolved profile of the concentration of the compound as a function of time within the epidermal layer, the dermal layer and the subcutaneous layer of the skin explant(s).

As mentioned above, FDC is not a suitable method to measure concentrations of a compound in the lower dermis or subcutis. At the same time, OFM probes are manually inserted in the skin explants, normally using a canula as a guide, and are very difficult to insert into the epidermis or the upper dermis. The combination of the two methods is therefore unique in that it allows to obtain a permeation profile across several layers of the skin, which neither of the two methods can provide on its own.

Thus, in a Second Aspect of the Invention, there is provided an in vitro method to evaluate diffusion of a compound into the skin, the method comprising:

(i) applying the compound to the stratum corneum of a first skin explant, determining the concentration of the compound within the upper dermis of the first skin explant at different time-points over a period of time using a Diffusion Cell (DC); and

(ii) applying the compound to the stratum corneum of a second skin explant, determining the concentration of the compound within the lower dermis and subcutaneous layer of a second skin explant at different time-points over a period of time using open flow micro-perfusion (OFM).

(iii) combining the concentrations determined in (i) and (ii) to generate a time-resolved profile of the concentration of the compound as a function of time within the upper dermis, lower dermis and subcutaneous layer of the skin explants.

Steps (i) and (ii) may be performed in any order, for example, sequentially or in parallel.

In some embodiments, the compound is diclofenac or salt thereof. Particularly, the compound is diclofenac sodium or diclofenac diethylammonium.

In a Third Aspect of the Invention, there is provided an in vitro method for comparing the effect of excipients on diffusion of a compound into the skin, comprising:

(i) applying a first formulation comprising the compound to the stratum corneum of a skin explant, determining the concentration of the compound within the upper dermis of the skin explant at different time-points over a period of time using a Diffusion Cell (DC);

(ii) applying the first formulation comprising the compound to the stratum corneum of a skin explant, determining the concentration of the compound within the lower dermis and subcutis of the skin explant at different time-points over a period of time using open flow micro-perfusion (OFM);

(iii) applying a second formulation comprising the compound to the stratum corneum of a skin explant, determining the concentration of the compound within the upper dermis of the skin explant at different time-points over a period of time using a Diffusion Cell (DC);

(iv) applying the second formulation comprising the compound to the stratum corneum of a skin explant, determining the concentration of the compound within the lower dermis and subcutis of the skin explant at different time-points over a period of time using open flow micro-perfusion (OFM);

(v) combining the concentrations determined in (i) and (ii) to generate a first time-resolved profile of the concentration of the compound as a function of time within the upper dermis, lower dermis and subcutis of the skin explants;

(vi) combining the concentrations determined in (ii) and (iii) to generate a second time-resolved profile of the concentration of the compound as a function of time within the upper dermis, lower dermis and subcutis of the skin explants; and

(vii) comparing the first and second time-resolved profiles;

wherein steps (i), (ii), (iii) and (iv) are performed in any order (sequentially or in parallel), step (v) is performed after both steps (i) and (ii) are completed, step (vi) is performed after both steps (iii) and (iv) are completed and wherein step (vii) is performed after steps (v) and (vi) are completed.

In some embodiments, the first and second formulations comprise different excipients. In some embodiments, the first and second formulations comprise different quantities or concentrations of the same excipients.

In certain embodiments, the method is an in vitro method for use in determining bioequivalence, wherein the compound is an API, and wherein one of the first or second formulations is a topical formulation comprising the API and the other of the first or second formulations is a test formulation comprising the API.

In a Fourth Aspect of the Invention, there is provided a method for determining bioequivalence of a first formulation comprising a compound and a second formulation comprising the compound, the method comprising:

(i) generating a time-resolved profile of concentration data for the compound as a function of time within the upper dermis, lower dermis and subcutis of a skin explant for the first formulation, wherein the concentration data is obtained from samples collected ex vivo using Franz Diffusion Cell and open flow micro-perfusion;

(ii) generating a time-resolved profile of concentration data for the compound as a function of time within the upper dermis, lower dermis and subcutis of a skin explant for the second formulation, wherein the concentration data is obtained from samples collected ex vivo using Franz Diffusion Cell and open flow micro-perfusion; and

(iii) comparing the first and second time-resolved profiles to determine bioequivalence.

Particularly, the first formulation comprises a compound, for example an API, and at least one pharmaceutically acceptable carrier, excipient or diluent. Particularly, the second formulation comprises a compound, for example an API, and at least one pharmaceutically acceptable carrier, excipient or diluent. Particularly, the first and second formulations comprise the same compound, for example, the same API. Particularly the concentration data is generated using the in vitro method of the First, Second or Third Aspects.

## DESCRIPTION OF DRAWINGS/FIGURES

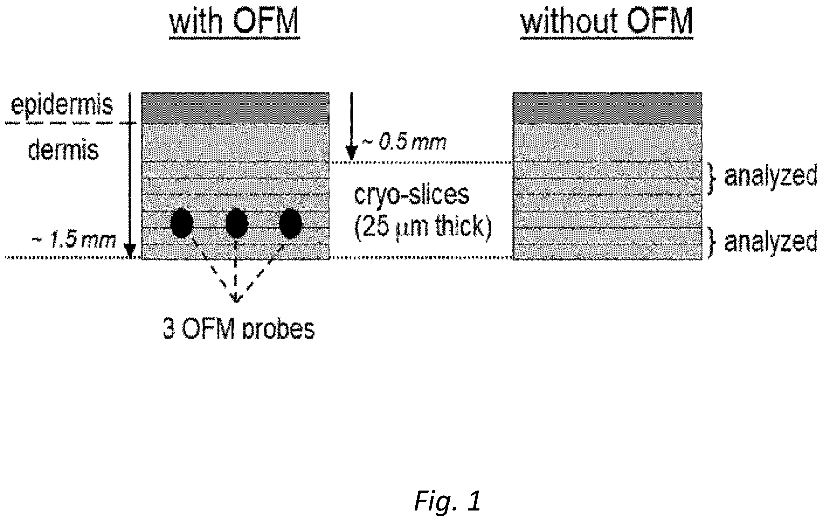

**FIGURE 1:** Schematic of the experimental set up for investigation of the influence of OFM sampling on the dermal concentration gradient of diclofenac. The scheme is not to scale and does not depict the exact number of analyzed tissue stacks.

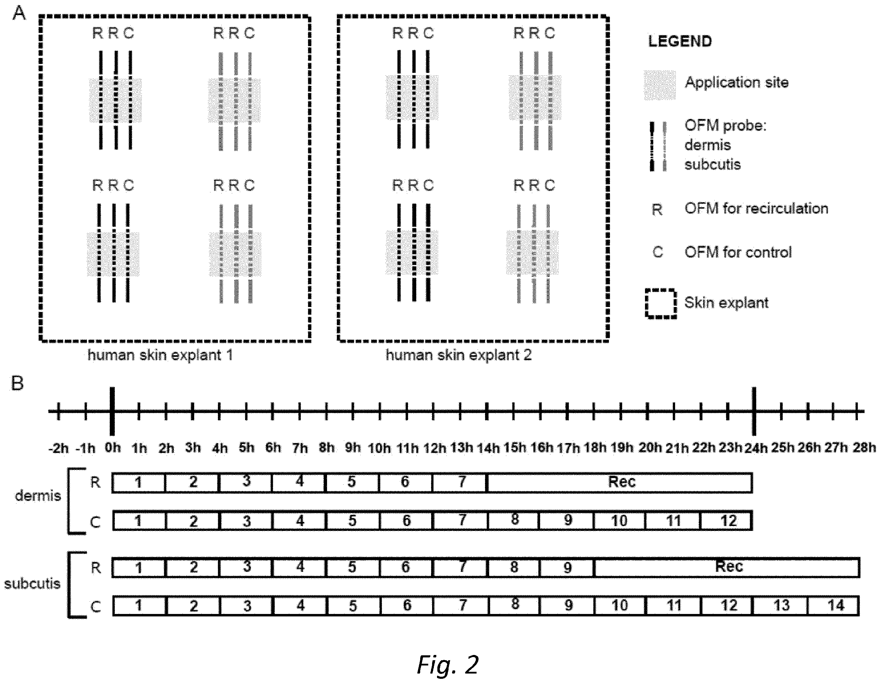

**FIGURE 2:** (A) schematic of the experimental setup used to investigate the relative recovery of diclofenac in dermal and adipose tissue; (B) timeline for collection of standard control OFM samples (diluted ISF) and OFM recirculation samples (undiluted ISF).

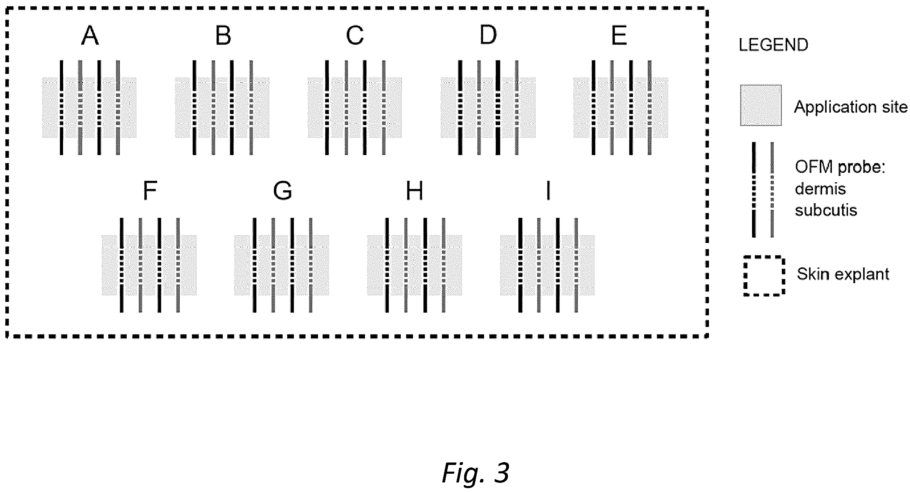

**FIGURE 3:** Schematic of the experimental setup used to investigate the time-resolved diclofenac concentration in dermis and subcutis.

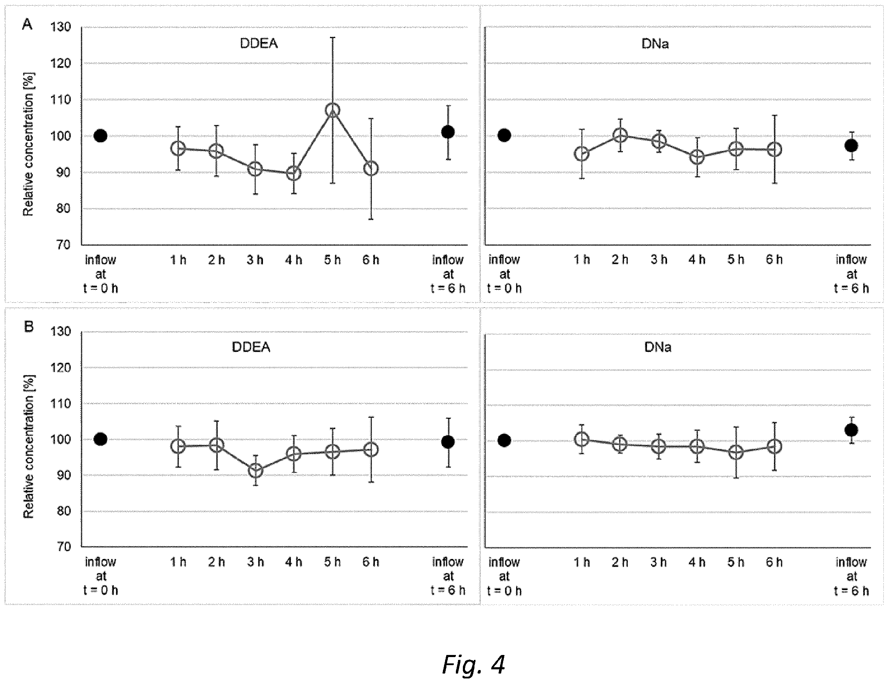

**FIGURE 4:** Relative DDEA and DNa concentration (open circles) as a function of recirculation time through (A) OFM probes and (B) OFM tubing. The concentration in the containers providing the inflow solutions (syringes in A or Eppendorf® vials in B) were measured at t =0 and 6 h and are shown as filled circles. The data are reported as mean $\pm$ SD percentage of the inflow diclofenac concentrations measured at t = 0 h (n = 6 for the OFM probes and tubing, and n = 2 for the syringes and Eppendorf® vials).

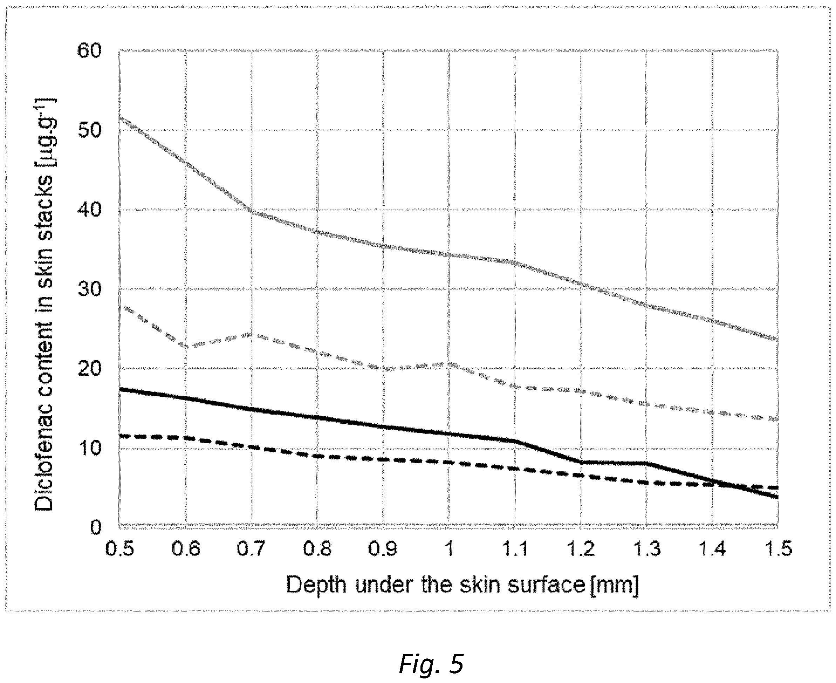

**FIGURE 5:** Diclofenac concentration in skin stacks as a function of depth in presence (dashed lines) and in absence (solid lines) of OFM sampling. The data represent geometric means of the concentrations measured using two skin donors (shown in grey and black for Donors 1 and 2, respectively). The number of stacks submitted or not to OFM sampling was two and three for Donor 1, and four and four for Donor 2, respectively.

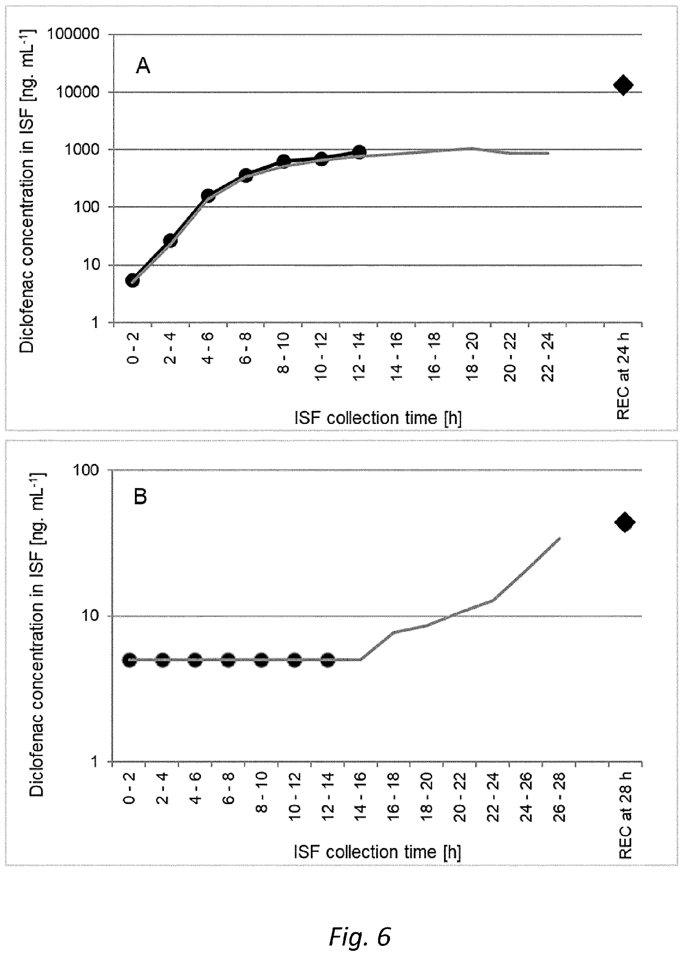

**FIGURE 6:** Diclofenac concentrations in the OFM probes run in standard sampling mode and used for control (grey lines) and recirculation (black circles) as a function of ISF collection time in the dermis (A) and subcutis (B). The data represent the geometric means of the individual measurements (n = 4 for control and n = 8 for recirculation in dermis and subcutis). REC (shown as diamonds) indicates the geometric mean concentration measured in the probes (n = 8 in dermis and in subcutis) used for recirculation at the end of sampling period (24 h in the dermis and 28 h in the subcutis).

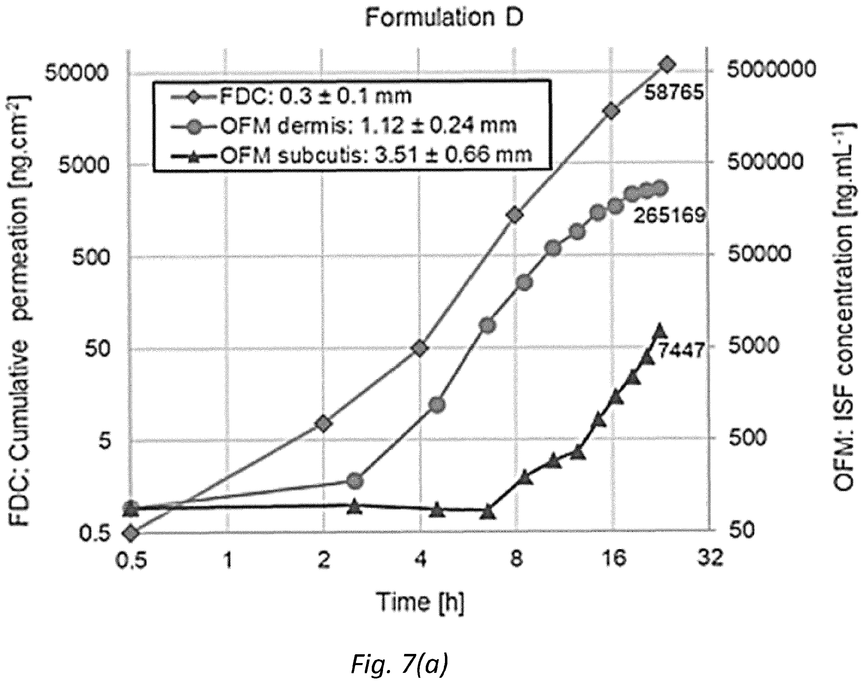

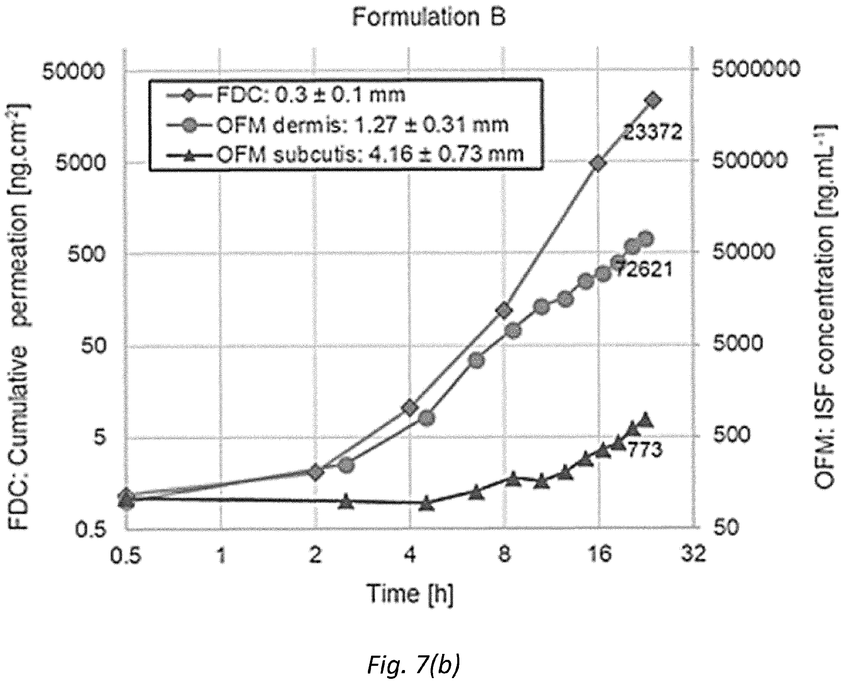

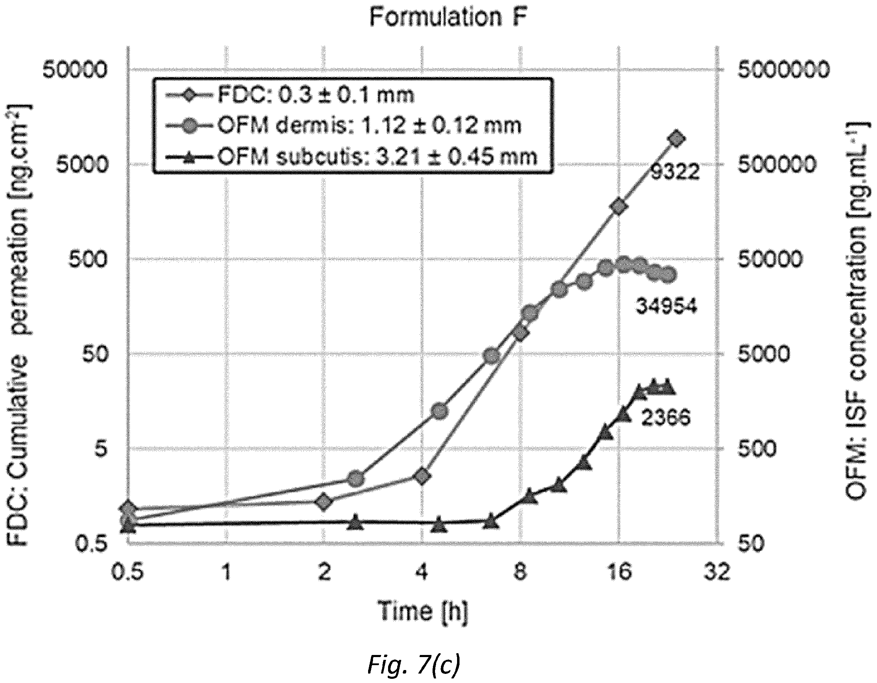

**FIGURE 7:** Time-resolved profiles of diclofenac cumulative permeation measured by FDC (left axis, diamonds) and ISF concentration measured by OFM (right axis) in the dermis (circles) and subcutis (triangles) following application of Formulations D (Fig. 7(a)), B (Fig. 7(b)), and F (Fig 7(c)). The symbols represent geometric means of the individual measured values (for FDC, n = 12 for Formulations D and F and n = 10 for Formulation B; for OFM, n = 12 for Formulations B and D and n = 10 for Formulation F), and the lines are for guidance only. The numbers in the legend indicate the average depth of sample collection (for FDC, corresponding to the average skin thickness, and for OFM - to the probe positions).

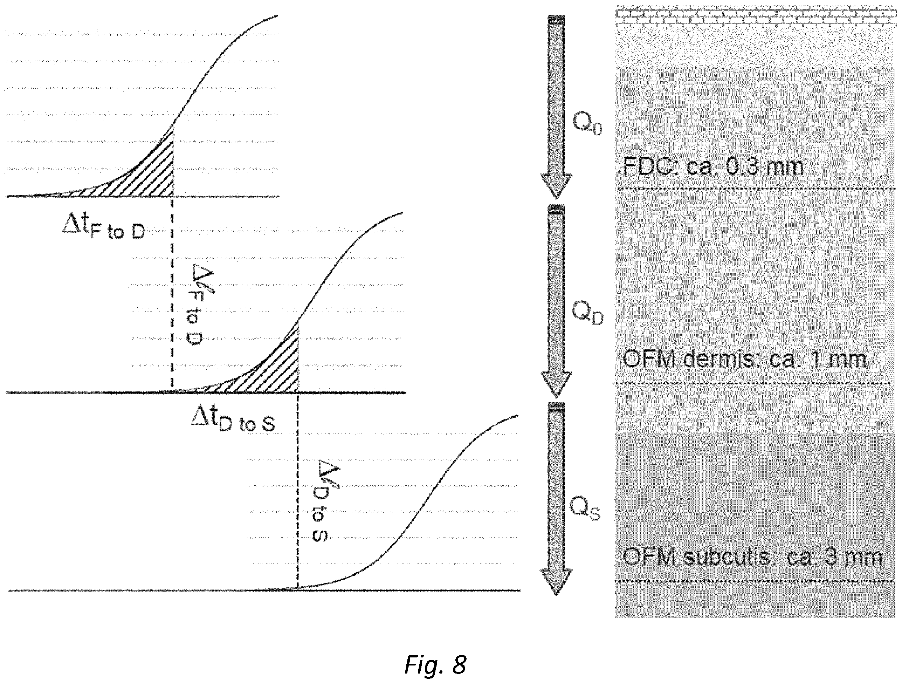

**FIGURE 8:** Schematic illustrating the proposed multi-compartmental model for diclofenac. The cartoon on the right shows the skin layers that were probed by the combined application of FDC and OFM, and the schemes on the left - the time courses observed by FDC (top) and OFM dermal (middle) and subcutaneous (bottom) ISF sampling. Note that the epidermis is shown in a lighter shade of grey, and the dermis in a darker shade of grey.

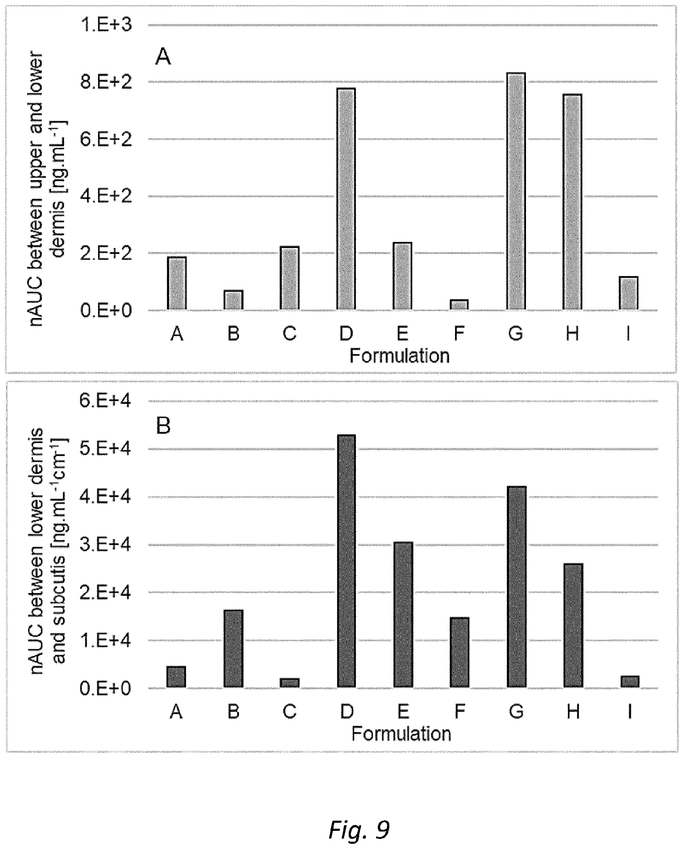

**FIGURE 9:** Driving gradients nAUC between upper and lower dermis (A) and between lower dermis and subcutis (B) calculated for the nine studied formulations by Eq. 2 using the geometric means of the individual repeats. For the FDC data, n = 12 for formulations D, F, G, H, and I, and n = 10 for formulations A, B, C, and E. For the OFM data, n = 12 for all formulations except F for which n = 10.

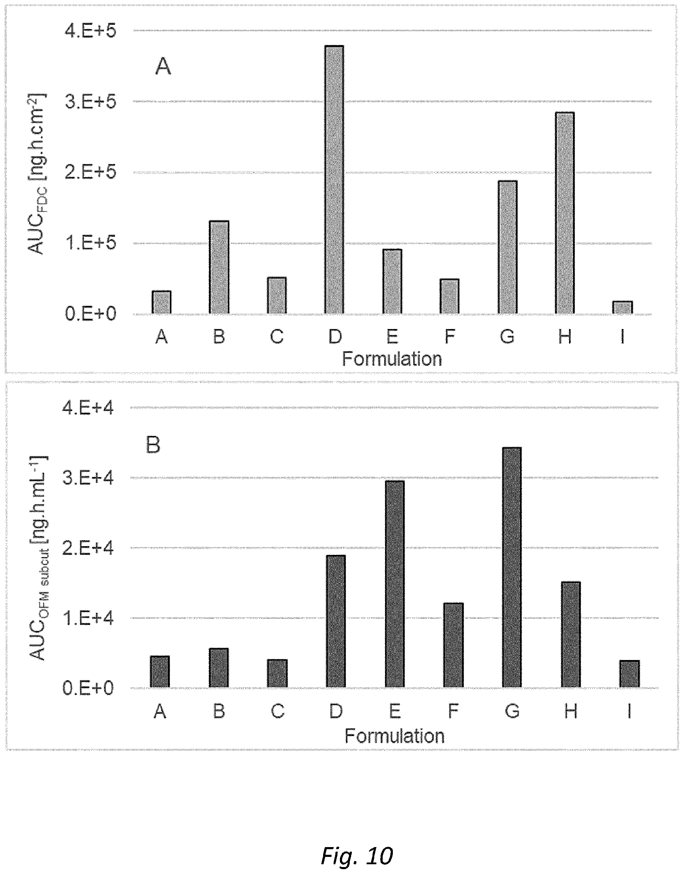

**FIGURE 10:** Driving gradients to reach the lower dermis (A) and to reach soft tissues underneath the subcutis (B) calculated for the nine studied formulations as AUC of the geometric means of the individual time courses of FDC cumulative absorption (A) and OFM subcutaneous ISF concentration (B). For the FDC data, n = 12 for formulations D, F, G, H, and I, and n = 10 for formulations A, B, C, and E. For the OFM data, n = 12 for all formulations except F for which n = 10.

## DETAILED DESCRIPTION OF THE INVENTION

Topical formulations for application to the skin can be useful in cosmetic applications or for transdermal administration of APIs. Transdermal administration of an API may be advantageous for a number of reasons, including avoiding first-pass metabolism, circumventing gastrointestinal absorption and targeting the API to a specific part of the body. However, despite such advantages, formulating topical formulations for transdermal administration of APIs is not always straightforward.

Structurally, the skin consists of a number of layers. The outermost layer is the epidermis. This is a relatively thin layer, the outermost surface of which is called the stratum corneum comprising flattened dead cells filled with keratin. The stratum corneum is highly impermeable, acting as a barrier. Beneath the epidermis is a dermal layer which comprises the upper dermis and lower dermis. Beneath the dermal layer is a subcutaneous layer also referred to as the subcutis or hypodermis.

To deliver a compound into or through the skin in a sufficient concentration often requires use of a formulation that reduces or overcomes the barrier action of the skin.

The Inventors have discovered that time-resolved permeation profiles of passive diffusion of compounds through the skin may be generated by combining data obtained using in vitro open flow micro-perfusion and Diffusion Cell apparatus, particularly a Franz diffusion cell (FDC). The combined data provides, for the first time, a complete picture of penetration and diffusion of a compound across the skin barrier and through the epidermal, upper dermal, lower dermal and subcutaneous layers of the skin. The methods of the invention may be used to identify excipients and other components that improve or enhance skin penetration and diffusion, enabling direct comparison and optimization of formulation candidates early in the development process by reliable selection progressing only those that exhibit enhanced passive diffusion through all skin layers.

The experimental data derived from the methods of the invention also greatly improve the required robustness and scope of application of mechanistic PBPK models supporting bioequivalence studies for topical products. Surprisingly, whilst the methods are reliable, simple and economic to perform, they have not previously been used in combination.

Thus, there is provided an in vitro method to evaluate diffusion of a compound into the skin which comprises:

(i) applying the compound to a surface of a first skin explant, determining the concentration of the compound within the first skin explant using a Diffusion Cell (DC); and
(ii) applying the compound to a surface of a second skin explant, determining the concentration of the compound within the second skin explant using open flow micro-perfusion (OFM).

The compound may be any active pharmaceutical ingredient (API). Particularly, the compound is part of a composition comprising the compound and at least one pharmaceutically acceptable carrier. More particularly, the compound is part of a composition formulated for topical application. A "topical formulation" refers to a composition that can be applied to the external surface of the skin of a subject. By way of non-limiting example, such formulations include emulsions, ointments, creams, pastes, gels and lotions.

The term "skin explant" as used herein refers to an ex vivo piece of skin tissue obtained from a suitable mammal, for example by excision during surgery. For DC, particularly FDC, the skin explant comprises at least the epidermis and upper dermis. For OFM the skin explant comprises the epidermis, dermis (upper and lower) and subcutaneous layers. The suitable mammal may be a mouse, guinea pig, rat, rabbit, dog, cat, pig, cow, sheep, goat, horse or human. Particularly, the suitable mammal is a human. More particularly, the skin explant is a human skin explant. For the avoidance of doubt, the methods of the invention are in vitro methods using ex vivo skin explants.

The compound to be tested, or formulation comprising the compound to be tested may be applied to the surface of the skin explant at any suitable dose. Particularly, the compound to be tested, or formulation comprising the compound to be tested is applied to the stratum corneum of the skin explant at a suitable dose. In some embodiments, the compound is diclofenac or salt thereof. Particularly, the compound is diclofenac sodium or diclofenac diethylammonium.

In some embodiments, a suitable dose (amount of formulation per square centimeter of skin explant) may be from 1mg/cm to 100mg/cm, particularly from 1mg/cm to 50mg/cm, more particularly from 1mg/cm to 25mg/cm, such as 1mg/cm, 5mg/cm, 10mg/cm, 15mg/cm or 20mg/cm.

In some embodiments, a suitable dose (amount of formulation per square centimeter of skin explant) may be from 1uL/cm to 100uL/cm, particularly from 1uL/cm to 50uL/cm, more particularly from 1uL/cm to 25uL/cm, such as 1uL/cm, 5uL/cm, 10uL/cm, 15uL/cm or 20uL/cm.

In some embodiments, a suitable dose (amount of compound per square centimeter of skin explant) may be from 0.1mg/cm to 100mg/cm, particularly from 0.1mg/cm to 50mg/cm, more particularly from 0.1mg/cm to 25mg/cm, such as 0.1mg/cm, 0.2mg/cm, 0.3mg/cm, 0.4mg/cm, 0.5mg/cm, 0.6mg/cm, 0.7mg/cm, 0.8mg/cm, 0.9mg/cm or 1mg/cm.

**Open flow micro-perfusion**

Open flow micro-perfusion (OFM) is a sampling method known in the art (see for example WO/2007/131780). OFM probes comprise a tube made of a flexible biocompatible impermeable material having a central section having macroscopic openings which, in use, allow for the exchange of substances between the tissue and a perfusion fluid circulating within the probe.

The present Invention uses OFM in vitro for continuous sampling of analytes from the interstitial fluid (ISF) of a skin explant.

Briefly, at least one OFM probe is inserted within the skin explant, parallel with the epidermal layer. The OFM probe is perfused with a solution (the perfusate), for example a physiological solution such as an electrolyte solution. Preferably the perfusate contains 1% HSA.

The skin explant comprises at least one test site. As used herein, the term "test site" refers to a distinct location on the skin explant where the compound or formulation to be tested is applied and within which at least one OFM probe is inserted for sampling. The first skin explant may comprise more than one test site, for example, two, three, four, five, six, seven, eight, nine, ten or more test sites.

In the methods of the Invention, at least one OFM probe is inserted within the lower dermis of the skin explant and/or within the subcutaneous layer of the skin explant. Preferably, at least one OFM probe is inserted into the lower dermis, and at least one OFM probe is inserted into the subcutis, and a test site comprises thus at least two OFM probes at different distances from the stratum corneum surface. A test site may comprise more than two OFM probes, for example, four OFM probes. In certain embodiments, each test site comprises four OFM probes, two within the dermis, particularly the lower dermis, and two within the subcutis. In certain embodiments, each test site comprises four OFM probes placed alternately in the dermis, particularly the lower dermis, and subcutis.

Before sampling, OFM probes may be flushed to remove any blood. Sampling is initiated by perfusing each OFM probe with the perfusate with a flow rate of from 0.1 to 10 µL/min. Particularly a flow rate of 1 µL/min.

OFM sampling of the interstitial fluid (ISF) from the dermis and subcutis may be performed at regular time-points following application of the compound to be tested or formulation comprising the compound to be tested (time=0 hours or T=0). For example, samples may be collected every 30 minutes for example at T=0, T=0.5, T=1.0, T=1.5, T=2.0, T=2.5, etc. Samples may be collected every hour, for example at T=0, T=1.0, T=2.0, T=3.0, T=4.0, T=5.0, etc. Particularly each sample is collected continuously over the course of a sampling period. For example, a first sampling period may start at T=0 and end with collection at T=1.0, a second sampling period may start at T=1.0 and end with collection at T=2.0, a third sampling period may start at T=2.0 and end with collection at T=3.0, etc. After each sampling period the sample vial is removed and a fresh sample vial attached to each OFM probe. Suitable sampling periods include 30 minutes or one hour (60 minutes). A sampling period of 30 minutes provides a temporal resolution of 30 minutes. A sampling period of one hour provides a temporal resolution of one hour.

The duration across which samples are collected may be referred to as the duration of the experiment. The duration of the experiment may be from 30 minutes to 24 hours, or more. In some embodiments the duration of the experiment is 12 hours. In some embodiments the duration of the experiment is 24 hours. In particular embodiments, the duration of the experiment is 24 hours and samples are collected continuously with a temporal resolution of one hour.

At the end of the experiment, the depth of each OFM probe may be measured.

Collected samples may be stored, for example, at -20 °C or -80 °C.

**Diffusion Cell**

Diffusion cells, such as the Franz Diffusion Cell (FDC), are known in the art (see for example US5547351 or US5198109) for measuring skin permeation and diffusion. Briefly, a diffusion cell comprises a donor compartment and a receiver compartment separated from each other by a membrane or a skin explant. The temperature of each compartment can be controlled. A skin explant is placed on the membrane or fixed between the donor and the receptor chamber with the stratum corneum placed upwards. The compound to be tested or formulation to be tested is applied to the stratum corneum. The receptor chamber comprises a solution that contacts the membrane and base of the skin explant. The solution is temperature controlled and stirred, for example, using a magnetic stirrer. The compound to be tested may diffuse through the skin explant into the solution in the receptor chamber. Samples of the solution may be taken from the receptor chamber via a sampling port using a micropipette or syringe. This step of the method may be conducted following the methodology provided in OECD Test Guideline No. 428 (2004) for skin penetration studies and the accompanying OECD Guidance Document No. 28.

The present Invention uses a diffusion cell in vitro for continuous sampling of analytes from a skin explant. Preferably the diffusion chamber is a Franz Diffusion Chamber. More preferably, the diffusion chamber is a static Franz Diffusion Chamber. In some embodiments, the Franz Diffusion Chamber is a static Franz Diffusion Chamber having a receptor chamber volume of 5mL. In some embodiments, the Franz Diffusion Chamber is a static Franz Diffusion Chamber having a receptor chamber volume of 10mL.

The skin explant used for DC, particularly FDC, comprises the epidermal layer and the upper dermis only. The skin explant may be a split thickness skin explant, for example, prepared by cutting the skin with an electric dermatome to a depth of 200 $\mu$m to 400 $\mu$m. Particularly the skin explant used for DC, particularly FDC comprises the epidermal layer and upper dermis but does not comprise the lower dermis or subcutis.

Preferably the skin explant has a resistance of at least 4 k$\Omega$ such as 4 k$\Omega$ or greater, such as 5 k$\Omega$ or greater, 6 k$\Omega$ or greater, 7 k$\Omega$ or greater, 8 k$\Omega$ or greater, 9 k$\Omega$ or greater, 10 k$\Omega$ or greater as measured by electrical resistance barrier integrity assessment.

The skin explant may have a skin surface area of from $0.5cm^2$ to about $3.5cm^2$, for example about $0.64cm^2$ or about $3.14cm^2$.

Particularly the receptor chamber contains receptor solution. Particularly, the receptor solution is phosphate buffered saline solution, more particularly phosphate buffered saline solution containing bovine serum albumin, yet more particularly phosphate buffered saline solution containing 5% w/v bovine serum albumin.

Sampling is performed by removing a volume of receptor solution from the receptor chamber and replenishing the receptor chamber with fresh receptor solution. The sample volume may be any suitable volume. Suitable volumes include a volume of from 50 $\mu$L to 500 $\mu$L, such as 50 $\mu$L, 100 $\mu$L, 150 $\mu$L, 200 $\mu$L, 250 $\mu$L, 300 $\mu$L or 350 $\mu$L. In certain embodiments the sample volume is 300 $\mu$L.

Sampling of the receptor solution may be performed at regular time-points following application of the compound to be tested or formulation comprising the compound to be tested (time=0 hours or T=0).

For example, samples may be collected every 30 minutes for example at T=0, T=0.5, T=1.0, T=1.5, T=2.0, T=2.5, etc. Samples may be collected every hour, for example at T=0, T=1.0, T=2.0, T=3.0, T=4.0, T=5.0, etc. In some embodiments, samples are collected at T=0, T=2, T=4, T=8, T=16 and T=24.

The duration of the experiment may be from 30 minutes to 24 hours, or more. In some embodiments the duration of the experiment is 12 hours. In some embodiments the duration of the experiment is 24 hours. In particular embodiments, the duration of the experiment is 24 hours and samples are collected at T=0, T=2, T=4, T=8, T=16 and T=24.

Collected samples may be stored, for example, at -20 °C or -80 °C.

## Sample analysis

The steps of the method comprise determining the concentration of the compound in the respective skin explants. Samples produced using OFM and DC, particularly FDC, are analysed to provide quantitative data on the compound to be tested, particularly data on the concentration of the compound to be tested is determined.

Samples may be analysed using a method suitable for analysing the compound to be tested. By way of non-limiting example, suitable methods include mass spectroscopy (MS), Liquid chromatography (LC) such as High-performance liquid chromatography (HPLC). A person skilled in the art will be able to select an appropriate analysis method for a compound of interest.

In some embodiments of the invention, the OFM samples may be analysed using mass spectroscopy, for example a mass spectrometer coupled to an HPLC system. In more specific embodiments, the OFM samples may be analysed using a Thermo LTQ Orbitrap XL mass spectrometer equipped with a heated electrospray ionization source coupled to the Thermo U3000 HPLC system.

In some embodiments of the invention, the receptor solution samples may be analysed using liquid chromatography and/or mass spectroscopy, for example using liquid chromatography-tandem mass spectrometry (LC MS/MS).

Preferably, the quantitative data is used to generate a time-resolved profile of the concentrations of the compound to be tested. The time-resolved profile shows the concentration of the compound to be tested as a function of time at a number of locations within the skin explant(s). Particularly, quantitative data generated by OFM and FDC are combined to provide a time-resolved profile of the concentration of the compound to be tested as a function of time within the upper dermis, the lower dermis and the subcutaneous layer of the skin explants.

Thus, the in vitro method to evaluate diffusion of a compound into the skin, may comprise:

(i) applying the compound to the stratum corneum of a first skin explant, determining the concentration of the compound within the first skin explant at different time-points over a period of time using a Diffusion Cell (DC); and

(ii) applying the compound to the stratum corneum of a second skin explant, determining the concentration of the compound within the second skin explant at different time-points over a period of time using open flow micro-perfusion (OFM).

(iii) combining the concentrations determined in (i) and (ii) to generate a time-resolved profile of the concentration of the compound as a function of time within the skin explants.

Steps (i) and (ii) may be performed in any order, for example, sequentially or in parallel.

In some embodiments, the in vitro method to evaluate diffusion of a compound into the skin, comprises:

(i) applying the compound to the stratum corneum of a first skin explant, determining the concentration of the compound within the upper dermis of the first skin explant at different time-points over a period of time using a Diffusion Cell (DC); and

(ii) applying the compound to the stratum corneum of a second skin explant, determining the concentration of the compound within the lower dermis and subcutis of the second skin explant at different time-points over a period of time using open flow micro-perfusion (OFM).

(iii) combining the concentrations determined in (i) and (ii) to generate a time-resolved profile of the concentration of the compound as a function of time within the upper dermis, lower dermis and subcutis of the skin explants.

Again, steps (i) and (ii) may be performed in any order, for example, sequentially or in parallel.

In some embodiments, the method is an in vitro method to compare the effect of excipients on diffusion of a compound into the skin, comprises:

(i) applying a first formulation comprising the compound to the stratum corneum of a skin explant, determining the concentration of the compound within the upper dermis of the skin explant at different time-points over a period of time using a Diffusion Cell (DC);

(ii) applying the first formulation comprising the compound to the stratum corneum of a skin explant, determining the concentration of the compound within the lower dermis and subcutis of the skin explant at different time-points over a period of time using open flow micro-perfusion (OFM);

(iii) applying a second formulation comprising the compound to the stratum corneum of a skin explant, determining the concentration of the compound within the upper dermis of the skin explant at different time-points over a period of time using a Diffusion Cell (DC);

(iv) applying the second formulation comprising the compound to the stratum corneum of a skin explant, determining the concentration of the compound within the lower dermis and subcutis of the skin explant at different time-points over a period of time using open flow micro-perfusion (OFM);

(v) combining the concentrations determined in (i) and (ii) to generate a first time-resolved profile of the concentration of the compound as a function of time within the upper dermis, lower dermis and subcutis of the skin explants;

(vi) combining the concentrations determined in (ii) and (iii) to generate a second time-resolved profile of the concentration of the compound as a function of time within the upper dermis, lower dermis and subcutis of the skin explants; and

(vii) comparing the first and second time-resolved profiles.

A person skilled in the art will understand that Steps (i), (ii), (iii) and (iv) may be performed in any order, for example, sequentially or in parallel. Steps (v) and (vi) may be performed in any order, either sequentially or in parallel subject to the proviso that step (v) is performed after both steps (i) and (ii) are completed and step (vi) is performed after both steps (iii) and (iv) are completed. Step (vii) is performed after steps (v) and (vi) are completed.

In some embodiments, the method is an in vitro method to compare bioequivalence comprising:

(i) applying a topical formulation comprising an API to the stratum corneum of a skin explant, determining the concentration of the API within the upper dermis of the skin explant at different time-points over a period of time using a Diffusion Cell (DC);

(ii) applying the topical formulation comprising an API to the stratum corneum of a skin explant, determining the concentration of the API within the lower dermis and subcutis of the skin explant at different time-points over a period of time using open flow micro-perfusion (OFM);

(iii) applying a test formulation comprising the API to the stratum corneum of a skin explant, determining the concentration of the API within the upper dermis of the skin explant at different time-points over a period of time using a Diffusion Cell (DC);

(iv) applying the test formulation comprising the API to the stratum corneum of a skin explant, determining the concentration of the API within the lower dermis and subcutis of the skin explant at different time-points over a period of time using open flow micro-perfusion (OFM);

(v) combining the concentrations determined in (i) and (ii) to generate a first time-resolved profile of the concentration of the API as a function of time within the upper dermis, lower dermis and subcutis of the skin explants;

(vi) combining the concentrations determined in (ii) and (iii) to generate a second time-resolved profile of the concentration of the API as a function of time within the upper dermis, lower dermis and subcutis of the skin explants; and

(vii) comparing the first and second time-resolved profiles to determine bioequivalence.

A person skilled in the art will understand that steps (i), (ii), (iii) and (iv) may be performed in any order, for example, sequentially or in parallel. Steps (v) and (vi) may be performed in any order, either sequentially or in parallel subject to the proviso that step (v) is performed after both steps (i) and (ii) are completed and step (vi) is performed after both steps (iii) and (iv) are completed. Step (vii) is performed after steps (v) and (vi) are completed.

In some embodiments, the method is an in vitro method to compare bioequivalence comprising:

(i) combining data relating to the concentration of an API as a function of time within the upper dermis, lower dermis and subcutis of a plurality of skin explants to generate a first time-resolved profile of the concentration of the API, wherein the data is generated from OFM and FDC sampling using a topical formulation comprising the API;

(ii) combining data relating to the concentration of an API as a function of time within the upper dermis, lower dermis and subcutis of a plurality of skin explants to generate a second time-resolved profile of the concentration of the API, wherein the data is generated from OFM and FDC sampling using a test formulation comprising the API; and

(iii) comparing the first and second time-resolved profiles to determine bioequivalence.

There is also provided a system for evaluating diffusion of a compound into the skin, the system comprising: a Franz Diffusion cell comprising a donor compartment and a receiver compartment and a first skin explant having an epidermal layer and an upper dermis layer wherein the skin explant is positioned between the donor compartment and the receiver compartment, and an open flow micro-perfusion (OFM) system and a second skin explant having an epidermal layer, upper dermis, lower dermis and subcutaneous layer wherein the OFM system comprises a first OFM probe and a second OFM probe implanted within the second skin explant substantially parallel to the epidermal layer.

Particularly, the first OFM probe is positioned within the lower dermis of the second skin explant and the second OFM probe is positioned within the subcutaneous layer of the second skin explant.

### General

The term "comprising" encompasses "including" e.g. a composition "comprising" X may include something additional e.g. X + Y. In some implementations, the term "comprising" refers to the inclusion of the indicated active agent, such as recited compounds, as well as inclusion of other active agents, and carriers, excipients, emollients, stabilizers, etc., known in the consumer health industry and generally recognized as safe (GRAS). Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as $x \pm 10\%$, $x \pm 5\%$, $x \pm 4\%$, $x \pm 3\%$, $x \pm 2\%$ or $x \pm 1\%$. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated.

All references or patent applications cited within this patent specification are incorporated by reference herein.

In order that this invention may be better understood, the following examples are set forth. These examples are for purposes of illustration only and are not to be construed as limiting the scope of the invention in any manner.

## EXAMPLES

Example 1

Nine test formulations were prepared, at dosing rates and regimens as summarized in Table 1.

*Table 1. Test formulations and their associated dosing rates and regimens.*

| Formulation and pharmaceutical form | Diclofenac salt concentration [% w/w, except D: % w/v] | No. of applications over 24h | Dose product per application [mg cm$^{-2}$, except D: $\mu$l cm$^{-2}$] | Diclofenac dose per application [mg cm$^{-2}$] | Total diclofenac dosed in 24h [mg cm$^{-2}$] |
|---|---|---|---|---|---|
| A: gel | 2% DNa | 2 (0, 12h) | 10 | 0.2 | 0.4 |
| B: solution | 2% DNa | 2 (0 12h) | 10 | 0.2 | 0.4 |
| C: gel | 1% DNa | 4 (0, 4, 8, 12h) | 10 | 0.1 | 0.4 |
| D: solution | 4.64% DDEA | 4 (0, 4, 8, 12h) | 2.5 | 0.1 | 0.4 |
| E: solution | 1% DNa | 1 (0 h) | 40 | 0.4 | 0.4 |
| F: solution | 2% DNa | 1 (0 h) | 20 | 0.4 | 0.4 |
| G: gel | 2% DNa | 1 (0 h) | 20 | 0.4 | 0.4 |
| H: gel | 3% DNa | 1 (0 h) | 13.3 | 0.4 | 0.4 |
| I: gel | 1.16% DDEA | 4 (0, 4, 8, 12h) | 10 | 0.1 | 0.4 |

For testing, the formulations were applied evenly over the stratum corneum (SC) surface of ex vivo skin explants using a positive displacement pipette.

*Human skin explants used in the OFM studies*

Fresh human skin explants were provided by the BioBank Graz of the Medical University Graz, Austria. The skin was donated by male and female patients aged 28 to 63 years old undergoing abdominal plastic surgery at the Department for Plastic, Aesthetic, and Reconstructive Surgery of the Medical University Graz. Donors provided written informed consent and the study was approved by the ethics committee of the Medical University Graz under the EC number 28-151 ex 15/16. The human skin explants were cleaned with gauze swabs and purified water and were subsequently stored frozen at -20 °C.

A trans-epidermal water loss barrier integrity assessment was performed on all application sites before and after acclimatization of the skin explants in the climate chamber by using an Aquaflux apparatus (AF200, Biox Ltd., United Kingdom). All samples met the predefined cutoff of 12 g m$^2$ h.

The skin explants were thawed at room temperature for 12 h before the start of the study. They were fixed on a plate of extruded polystyrene wrapped in a medical underpad (MoliNea N, Paul Hartmann AG, Austria) and placed into a climate chamber (32 °C / 40-60% relative humidity) for 1 h before use. Ambient temperature and ambient relative humidity within the climate chamber were controlled throughout the experiment with a data logger with integrated temperature sensor and relative humidity sensor (175 H1 temperature and humidity data logger, Testo, Germany).

*Split-thickness human skin used in the FDC studies*

Seven samples of full thickness human abdominal skin were obtained from female donors aged 31 to 64 years old from NHS GG&C Bio-Repository, Glasgow (four samples), St John's Hospital, Livingston, NHS Lothian (one sample), and Tissue Solutions Ltd., Glasgow, UK (two samples). The skin was stored in a freezer set to maintain a temperature of -20 °C. The patients gave full consent for their tissues to be used and all suppliers followed their own local ethics approval for the collection of tissues. The split thickness skin was prepared by cutting the skin to a depth of 200-400 $\mu$m with an electric dermatome.

**Example 2: open flow micro-perfusion (OFM)**

*Non-specific adsorption of diclofenac on the OFM probes and tubing*

Two test solutions of diclofenac diethyl amine salt (DDEA) and diclofenac sodium salt (DNa) were tested. They comprised an electrolyte solution (ELO-MEL isotone, Fresenius Kabi, Austria) of 1% human serum albumin (HSA; Albunorm®, Octapharma, Austria) and 1 $\mu$g mL$^{-1}$ DDEA or DNa.

For each test solution, six OFM outlet sections (half of OFM-POX-15, JR-HEALTH, Austria) and six sections of OFM tubing (OFM-PS3-75, JR-HEALTH, Austria), were tested for 6 h. Samples were collected in 1 h intervals. Syringes and Eppendorf® vials were used to deliver test solution to the OFM outlet sections and tubing, respectively. Samples from the syringes and from the Eppendorf® vials were taken at the start (t = 0 h) and at the end (t = 6 h) of the experiment to determine the concentration entering the OFM probe and tubing. Syringes, dosing needles, Eppendorf® vials, and pipette tips were coated with each test solution prior to use. For coating, the samples were filled with test solutions and maintained at room temperature for 30 min. Afterwards, the coating test solutions were discarded. Pipette tips underwent three cycles of filling and emptying of test solutions. The fourth filling of the pipette tip was used as the sample. Adsorption was assessed by comparing inflow to outflow concentrations.

*OFM sampling*

Cannulas (Sterican 20G, B.Braun, Germany) were inserted into the dermis and the subcutis of the skin explant. OFM probes (OFM-POX-15, JR-HEALTH, Austria) were pushed through the cannulas. Afterwards, the cannulas were removed and the probes were fixed to the skin with cyanoacrylate (Cyanolit 241F, Panacol-Elosol GmbH, Germany).

Push and pull tubing (OFM-PS3-75, JR-HEALTH, Austria) were mounted into the OFM pump (MPP102, JR-HEALTH, Austria) and connected to the OFM probes and perfusate bag (OFM - BAG 10 mL, JR-HEALTH, Austria). The perfusate bag was filled with electrolyte solution (ELO-MEL isotone, Fresenius Kabi, Austria) containing 1% HSA (Albunorm®). The OFM probes were flushed to remove any blood and sampling was initiated with a flow rate of 1 $\mu$L min$^{-1}$ by connecting a sampling vial (0.2 mL PCR tubes, MAXYMUM Recovery). After each 1 h sampling period, the full sample vial was stored at -20 °C and a fresh vial was attached to the OFM probes. Samples were collected continuously for 24 h with a temporal resolution of 1 h. At the end of the experiment, all samples were stored at -80 °C.

*Influence of OFM sampling on the diclofenac concentration gradient within the skin*

The study was performed on thawed human skin explants from two donors. The explant from Donor 1 contained five application sites: three were used with OFM sampling and two without it. The explant from Donor 2 contained eight application sites: four were used with OFM sampling and four without it. Formulation A was applied at a dose of 10 mg cm$^{-2}$ to all application sites. Dermal OFM sampling was performed for 24 h with three OFM probes per application site. After 24 h, the depth of the OFM probes was measured and biopsies were collected from all application sites for dermal cryo-slicing. The depth of the OFM probes was measured using the high-frequency US LOGIQ e R6 (GE Healthcare, United Kingdom) with a 22 MHz transducer and US transmission gel (Aquasonic 100, Parker Laboratories Inc., USA) at the end of the OFM sampling. The mean depth of the probes was calculated by dividing the area between skin surface and upper side of the OFM probe by the US picture length.

For dermal cryo-slicing, residual topical test product was removed with tape stripping. Tape stripping was conducted with ten pieces of tape (Leukoplast Waterproof, BSN medical, Germany) *per* application site. Subsequently, an aluminum disc (30 mm diameter, JR-HEALTH) was glued to the skin with cyanoacrylate (Cyanolit 732F, Panacol, Germany). Skin was excised with a single-use scalpel. The disc with the attached skin was frozen at -20 °C and then transferred to -80 °C storage.

The skin was sliced parallel to the skin surface from the inner skin layers towards the superficial ones (Figure 1) with a cryotome (Cryostat-Microtome HM560M, Microm). Slicing commenced at a depth of 1.5 mm below the skin surface and was performed in 25 $\mu$m thick sections. Each pair of consecutive slices were stored in the same cryotube (CryoPure 1.8 mL, Sarstedt, Germany), resulting in one sample covering a depth of 50 $\mu$m. Samples were stored at -80 °C until bioanalysis. Diclofenac concentration was measured in every second sample resulting in a final spatial resolution of 100 $\mu$m.

*Determination of recovery (OFM dilution factor)*

Formulation A was applied at a dose of 10 mg cm$^{-2}$ on eight application sites using skin explants from two donors (Figure 2). Two sites from each explant were used to sample the dermis, and two were used to sample the subcutis. Three OFM probes were placed in each application site, at a depth of *ca.* 0.8 mm and *ca.* 3 mm below the skin surface to sample ISF in the dermis and subcutis, respectively.

Two OFM probes at each application site (indicated by R in Figure 2) were run first in standard OFM sampling mode and were then switched into recirculation OFM mode. During recirculation, the perfusate was circulated in a closed-loop until the analyte was at equilibrium between perfusate and ISF of the skin explant (Hummer et al. 2020). For switching into recirculation mode, the OFM pump was stopped and the OFM tubing was connected to form a closed loop. The pump was restarted and the perfusate was pumped inside the closed OFM system at 1 $\mu$L min$^{-1}$ for 10 h. The closed system contained 30 $\mu$L of perfusate, resulting in 20 recirculations in 10 h. After 10 h, the closed tubing system was opened, and the recirculation sample was collected.

The optimal time period to determine the relative recovery is during stable analyte concentration in tissue. For dermal sampling, recirculation OFM was performed between 14 and 24 h post product application; the subcutis sampling was expanded to cover the period between 18 and 28 h post product application, to allow for appropriate equilibration at this skin depth.

The third OFM probe in each application site (indicated by C in Figure 2) was used for standard OFM sampling and provided diluted ISF samples for a concentration-time profile. Thus, it served as a control to verify that a stable diclofenac concentration was reached during the recirculation phases.

*Collection of time-resolved profiles of diclofenac concentration by OFM*

The study was performed on six explants, each from a different donor. Each skin explant contained nine test sites for OFM sampling (Figure 3). Each test site contained four OFM probes, placed alternately in the dermis and subcutis. OFM sampling was performed hourly for 24 h and the depth of the OFM probes was measured thereafter as described above. Diclofenac concentration was analyzed only in samples with uneven numbers (1 h, 3 h, ..., 23 h).

*Bioanalysis of OFM samples*

DDEA and DNa were analyzed using a Thermo LTQ Orbitrap XL mass spectrometer equipped with a heated electrospray ionization source coupled to the Thermo U3000 HPLC system. Mass spectrometer detection was conducted in negative mode, using a full scan in the range of 250-400 m/z. Diclofenac sodium d6 was used as an internal standard for the quantification of diclofenac in the samples. LLOQ was 10 ng mL$^{-1}$.

OFM samples were spiked with internal standard (diclofenac sodium d6) and 5% formic acid. Samples were then applied to HLB SPE (hydrophilic-lipophilic balance solid phase extraction) cartridge (Waters Ges.m.b.H, Vienna, Austria). After washing, the sample was eluted with acetonitrile and was taken up in 30% acetonitrile.

**Example 3: Franz Diffusion Cell**

The experiments were conducted based on OECD Test Guideline No. 428 (2004) for skin penetration studies and the accompanying OECD Guidance Document No. 28.

The split-thickness skin was mounted in static diffusion cells. Phosphate buffered saline containing bovine serum albumin (5%, w/v) was used as the receptor fluid. The receptor fluid was degassed by sonication and stored at a temperature of *ca.* 4 °C prior to use. The receptor chamber volume was nominally 5 mL with a skin surface area 0.64 cm$^2$ except for Formulation D where these were 10 mL and 3.14 cm$^2$, respectively. The cells were positioned in a manifold heated to maintain a skin surface temperature of 32 $\pm$ 1 °C and the receptor fluid volume made up to the pre-calibrated line. The receptor fluid was mixed using a magnetic stirrer flea which was placed in the receptor chamber. An electrical resistance barrier integrity assessment was performed before product application; any skin sample exhibiting resistance lower than 10.9 k$\Omega$ (for the 0.64 cm$^2$ cells) or 4 k$\Omega$ (for the 3.14 cm$^2$ cells) was excluded from subsequent absorption measurements.

Before use, the study formulations containing diclofenac (see Table 1) were stored at ambient temperature. They were applied evenly over the SC surface of the skin using a positive displacement pipette at application rates and dosing times as described in the Table.

Absorption was assessed by collecting receptor fluid samples (300 $\mu$L) at 0 h (pre dose), 2, 4, 8, 16 and 24 h post dose. The removed receptor fluid volume (300 $\mu$L) was replenished with fresh receptor fluid solution after each withdrawal, except for the 24 h collection. At 24 h, following the receptor fluid collection, the cells were dismantled, and the skin samples were destroyed by incineration.

The receptor fluid samples were analyzed by a validated analytical method using liquid chromatography-tandem mass spectrometry (LC-MS/MS). The LLOQ was 1 ng mL$^{-1}$.

**Example 3: Data analysis and Results**

To enable the use of log-transformed statistics, in both the FDC and OFM datasets we imputed BLOQ values as 0.5 x LLOQ, namely as 5 ng mL$^{-1}$ for OFM and 0.5 ng mL$^{-1}$ for the FDC.

The OFM probe depth was calculated as the average of the individual 12 probe depths determined for each formulation, except for the subcutaneous probes for Formulation F; for those, the average probe depth was calculated over ten probes as the positions of two probes differed considerably from those across all formulations.

Before further analysis, the ISF concentrations measured by OFM were corrected for recovery using the dilution factor determined previously and represented as the geometric means of 12 samples *per* formulation. Only the dataset for the subcutaneous ISF content for Formulation F was n = 10 (see above).

For the FDC dataset, data collected from skin samples having log-transformed geometric mean TEER deviating by ± 2 x SD from the mean were excluded from further analysis. For Formulation B, the data from two individual FDC repeats were incomplete and were also excluded. Thus, the datasets for formulations D, F, G, H, and I were n = 12, and those from formulations A, B, C, E were n = 10.

*Non-specific adsorption of diclofenac on the OFM probes and tubing*

Figure 4 shows the concentration of diclofenac recovered after a 6 h perfusion of concentrated solutions of DDEA and DNa containing 1% BSA through the OFM probes (Figure 4A) and tubing (Figure 4B). It remained stable throughout the whole period, illustrating the absence of adsorption of DDEA and DNa to the OFM probes (one-tailed paired t-test "control start" to "1 h": DEA p = 0.111, Na p = 0.061) or tubing material (one-tailed paired t-test "control start" to "1 h": DEA p = 0.212, Na p = 0.422). Furthermore, DDEA and DNa were stable in the stock solution for the duration of the experiment (data not shown). Neither salt adsorbed to the syringes (one-tailed paired t-test "stock" to "syringe start" p = 0.365) or the Eppendorf® vials used for outflow collection as illustrated by the stable concentrations maintained throughout the experiment (filled circles in Figure 4).

These data confirmed that the precaution of adding 1% HSA to the perfusate suffices to prevent possible recovery losses of the drug due to adsorption to the OFM setup. Diclofenac exhibits extremely high protein binding (>99.8%). The protein concentration was chosen to mimic the concentration of albumin in the ISF of dermal and adipose tissue. Keeping in mind also the fairly low albumin-to-diclofenac molar ratios in this test (56:1 and 48:1 for DDEA and DNa, respectively) designed to match the worst-case scenario expected in the OFM work, and the fact that the protein offers up to 9 binding sites, the risk of adsorption losses during the *in vitro* human skin study can be considered as extremely low.

*Influence of OFM sampling on the diclofenac concentration gradient within the skin*

This part of the study was designed to test if the continuous sink condition caused by the OFM probe could potentially deplete the tissue and thereby affect the PK of diclofenac in *in vitro* skin after topical application. We analyzed a total of 13 skin stacks from two skin donors covering the depth of 0.5-1.5 mm. If the OFM sampling affected the tissue diclofenac concentrations, one would expect to observe a difference between the concentrations measured with and without OFM sampling, minimal at the skin surface (where the concentration is driven by the API release from the formulation and by its adsorption through the SC) and largest at or below the depth at which OFM probes were positioned (on average, 0.97 ± 0.25 mm below the skin surface).

Figure 5 shows the geometric mean of the diclofenac concentration as a function of depth for each donor. The largest difference between the concentrations measured in presence and absence of OFM sampling was found close to the skin surface at *ca.* 0.5 mm; it decreased in the deeper tissue. Furthermore, the presence of the OFM probes did not lead to a change of the slope of the concentration gradient. These results indicated that the observed differences in diclofenac concentration were due to intra-donor site-to-site variability of the skin and not to the OFM sampling. Apparently, OFM sampling did not perturb the drug concentration gradient within the skin.

*Determination of recovery (OFM dilution factor)*

Figure 6 shows the diclofenac concentrations in the OFM probes used for control and recirculation as a function of ISF collection time in the dermis (Figure 6A) and subcutis (Figure 6B).

In the dermis (Figure 6A), the DNa concentration in the control probes reached a stable plateau *ca.* 12 h after product application. The DNa concentration in the OFM probes dedicated for recirculation showed the same time course until 14 h when they were switched to recirculation mode for the remaining 10 h of the experiment. At the end of the experiment (24 h), the concentration measured in the recirculation probe (shown as a diamond) was considerably higher than the one in the control probes.

To calculate the dilution factor in the dermis, the concentration of diclofenac in the recirculation probe at the end of recirculation (i.e., at 24 h) was compared to the one observed in same probe just before the start of recirculation (i.e., in the time interval of 12-14 h post product application). This approach can be used when the drug concentration before the start of recirculation has reached a stable plateau - a condition that was met (Figure 6A). Under these conditions, the drug concentration measured before the start of recirculation corresponds to the one of diluted ISF, and the one measured in the recirculated sample corresponds to the one of undiluted ISF. The ratio between the two values gave a mean relative recovery of 7.2 $\pm$ 1.6% (corresponding to a dilution factor of 13.9-fold).

In the subcutis (Figure 6B), the DNa concentration measured in all probes (i.e., the ones used in standard OFM mode, and the ones switched to recirculation mode between 14 and 28 h post product application) was lower than the LLOQ until 14 h post product application. In the control probes, it did not reach a stable plateau throughout the whole measurement period of 28 h. At the end of the experiment (28 h), the DNa concentration measured in the recirculation probes was higher than the one in the control probes by only 9 ng mL$^{-1}$ (compare with the order of magnitude difference observed in the dermis).

The observed concentration time courses in the subcutis precluded the calculation of recovery. The concentration in both the probes used for recirculation and the control probes did not reach a stable plateau, and the concentration in the control probes increased considerably between 22 and 28 h. Apparently, even the extended overall duration of the experiment to 28 h (from the 24 h used in for the dermis) did not suffice to allow equilibration of the drug concentration sampled in the subcutis in either the control or the recirculation samples. Thus, in the subsequent data treatment the dilution factor calculated for the dermis was used; this conservative estimation precluded overestimating the diclofenac concentrations measured in the subcutis in the following studies.

*Time-resolved profiles of diclofenac concentration measured by FDC and OFM*

Figure 7 shows representative examples of the 24 h time-resolved profiles of diclofenac cumulative permeation and ISF concentration measured respectively by FDC and OFM following application of three topical formulations of different qualitative and quantitative composition (corresponding to formulations D, B, and F of Table 1). As detailed in the Table, they differed also in the diclofenac salt and its concentration (4.64% DDEA in D, and 2% DNa in B and F), and dosing regimen (four, two, and one application *per* day for D, B, and F, respectively). In both the FDC and OFM experiments, however, adaptation of the formulation quantity dosed *per* application event ensured that all products delivered the same 24 h total amount of 0.4 mg diclofenac *per* cm$^2$.

As expected from the differences highlighted above, the three formulations delivered very different time courses in the FDC in line with the well-known influence of the overall formulation composition, properties, and dosing regimen on the delivery of diclofenac across the SC barrier. The time courses collected *via* OFM also differed considerably, both in the dermis and the subcutis. These data clearly demonstrated that the influence of the formulation does not end with crossing the skin barrier; it continues to have a major impact on drug passive diffusion and distribution throughout the underlying soft tissues.

It was also explored whether the extent of diclofenac delivery to the dermis and subcutis (as measured by OFM) correlates with the delivery across the SC into the upper dermis (as measured by the FDC). To enable this comparison, the depth reached by diclofenac in the upper dermis was approximated by the thickness of the skin samples used in the FDC, and the depth reached in the lower dermis and subcutis was approximated by the position of the OFM probes in these layers. The API quantities measured using both *in vitro* methods correspond to the sum of those available for passive diffusion and those available for systemic absorption at the 24 h timepoint. The API downstream distribution under real-life, in-use conditions are further complicated by the eventual release of any API amounts bound to or retained inside the tissues that could become available for further redistribution with time, e.g., upon repeated product application or washing.

A top line visual inspection of the characteristics of the timecourses collected in the lower dermis and subcutis (e.g., lag time, maximal diclofenac concentration reached, slope, presence of a plateau) shows that they do not match those measured from the same products using the FDC; they also differ considerably among the three formulations. These data show that the passive diffusion of diclofenac through the dermis and subcutis does not correlate with its diffusion through the skin barrier (and the upper dermis) and cannot be predicted using FDC data alone. The results demonstrate that OFM *in vitro* provides valuable information on API concentrations available for passive diffusion through lower dermis and subcutis and for systemic uptake, complementing the data obtained *via* the classical FDC.

An initial, multi-compartmental approach to the analysis and comparison of the excipient-dependent passive diffusion of diclofenac through the skin layers was developed (Figure 8). Its structure incorporates also the deep dermis and the subcutis.

Once diclofenac has crossed the SC, it partitions sequentially into the upper dermis (and/or into the systemic circulation), the lower dermis, and the subcutaneous layer. The FDC receptor fluid concentrations (for a given exposure area) at any given timepoint represent the drug amounts that have reached a depth of 0.3 mm in the upper dermis (determined by the thickness of the skin samples used for FDC), and the FDC lag time corresponds to the timepoint at which diclofenac first reaches this depth.

Similarly, the time post application needed for diclofenac to first appear in the lower dermis ($\Delta t_{F \, to \, D}$ in Figure 8) is estimated by its first detection in the OFM dermal probes; the diffusion pathway to reach the lower dermis ($\Delta l_{F \, to \, D}$ in Figure 8) is estimated by the difference between the FDC skin thickness and the position of the OFM dermal probe in each individual sample. Likewise, the time post application needed for diclofenac to first appear in the subcutis ($\Delta t_{D \, to \, S}$ in Figure 8) is estimated by its first detection in the OFM subcutaneous probes; the diffusion pathway to reach the subcutis ($\Delta l_{D \, to \, S}$ in Figure 8) is estimated by the difference between the positions of the OFM dermal and subcutaneous probes in each individual sample. The passive diffusion fluxes of diclofenac to reach each of the three detection positions ($Q_o$, $Q_D$, and $Q_s$ for the upper dermis, lower dermis, and subcutis, respectively) are calculated as:

$$\text{Eq. (1):} \qquad\qquad Q = D \times K \times \Delta C / \Delta l,$$

where D is the diffusion coefficient, K is the partition coefficient, $\Delta C$ is the change in drug concentration between two skin layers, and $\Delta l$ is the diffusion pathlength. Thus, for a given formulation $\Delta C / \Delta l$ is the driving force through a given skin layer (i.e., between upper and lower dermis, and between lower dermis and subcutis). It can be calculated as the AUC in the upper layer required to initiate a flux into the lower layer (shown by the hashed sections in the time courses in Figure 8), normalized by $\Delta t$ and $\Delta l$ (denoted further as nAUC), or:

$$\text{Eq. (2):} \qquad\qquad \Delta C / \Delta l \ = AUC / (\Delta t \, \Delta l ) = nAUC.$$

It is well known that the formulation composition influences the diffusion flux $Q_o$ mainly through impacting the SC and that D and K for the same API differ between formulations. The influence of the formulation composition on D and K (and thereby, on $Q_D$ and $Q_s$) in the dermis and subcutis, however, is less well understood, even though the weight of evidence for such impact is considerable and growing. It is not clear whether a given combination of excipients impacts the transport of APIs through skin barrier, dermis, and subcutis similarly or differently, or whether the impact on the transport through one layer is predictive for the transport through another one.

These questions were probed by comparing the nAUC needed in an upper layer to initiate a flux into the adjacent lower layer (i.e., upper to lower dermis, and lower dermis to subcutis) observed for the nine studied formulations. A difference in the nAUC between formulations within the same skin layer would indicate that the composition impacts not only the drug delivery across the SC but also further downstream; similarly, a different ranking of a formulation in the different skin layers would indicate that its composition influences K and D in the dermis and subcutis differently. Of course, this approach can be applied only to compare the formulation ranking in terms of maximizing API delivery into the different skin layers but not to compare the numerical values obtained using the two techniques: this inherent limitation is imposed by the different units of nAUC measured in FDC (mass.volume$^{-1}$) and OFM (mass.volume$^{-1}$ length$^{-1}$).

For both the OFM and FDC data, a sigmoidal function was fitted through the individual measured data points plotted against time and AUC was computed between the timepoints described above using trapezoidal integration (Igor Pro 8.02, Wavemetrics). The first occurrence of a non-zero diclofenac concentration (i.e., greater than LLOQ) was estimated graphically from the sigmoidal fits.

Figure 9 compares the nAUC between upper and lower dermis (A) and between lower dermis and subcutis (B) among the formulations.

Within each skin layer, the nAUC varied considerably between formulations, evidencing the significant effect of their compositions on diclofenac passive diffusion through the entire dermis and subcutis. A broadly similar relative ranking of the formulations was observed in both dermis and subcutis, suggesting that the effect of the composition on the diffusion and partition coefficients of diclofenac in the two skin layers was similar.

It was also probed whether diclofenac diffusion across the skin barrier is representative of the diffusion-driven subcutaneous drug levels that would be available for further distribution into the underlying soft tissues (e.g., muscle). To this end, the driving gradient to reach the lower dermis $AUC_{FDC}$ (calculated as the 24 h cumulative absorption in FDC) and the driving gradient to reach soft tissues underneath the subcutis $AUC_{OFM \, subcut}$ (calculated as the 21 h cumulative diclofenac concentration of the subcutis ISF in OFM) were compared. Figure 10 shows this comparison.

The relative ranking of the formulations by their ability to deliver diclofenac across the skin barrier (A) and below the subcutis (B) differed considerably, evidencing that the API diffusion across the skin barrier is not predictive of its diffusion-driven subcutaneous ISF levels. Thus, for example, formulation D achieved the highest 24 h cumulative absorption across the SC, 2x higher than formulation G (Figure 10A). Formulation G, however, achieved the highest 24 h cumulative subcutaneous ISF levels, 1.8x higher than Formulation D (Figure 10B). Apparently, the different combinations of excipients in the two formulations have modulated in a different way D and K for diclofenac diffusion across the SC and in the subcutaneous layer.

This example illustrates the usefulness of applying the combination of methods to inform lead candidate selection: depending on the targeted site of action of an API having certain physicochemical properties, one or the other of these two compositions may be a better choice for progression during the product development process.

**Conclusion**

These Examples demonstrate the utility of applying in vitro OFM in conjunction with FDC. In the early stages of product development, using this combination of methods can help select formulation candidates that optimize the API passive diffusion not only across the skin barrier (as classically done using FDC) but also through the underlying full-thickness dermis and subcutis.

Such an expansion of the preclinical toolbox offers the benefit to "fail early, fail cheap" during topical product development, in the interest of the consumers and patients in need of efficacious and safe topical products. Using appropriately designed model formulations, this approach could also drive forth the understanding of the influence of specific excipients, and excipient combinations, on the diffusion and partition coefficients for a given API.

In view of this guidance, one of ordinary skill in the art will be able to adjust the parameters provided in the Examples to achieve similar results within the remit of the claims.

**Claims**

1. An in vitro method to evaluate diffusion of a compound into the skin which comprises:

   (i) applying the compound to a surface of a first skin explant, determining the concentration of the compound within the first skin explant using a Diffusion Cell; and
   (ii) applying the compound to a surface of a second skin explant, determining the concentration of the compound within the second skin explant using open flow micro-perfusion (OFM).

2. The in vitro method of Claim 1 wherein the open flow micro-perfusion utilises a perfusate comprising 1% human serum albumin.

3. The in vitro method of Claim 1 or 2, wherein the Diffusion Cell apparatus is a Franz Diffusion Cell (FDC), particularly a static Franz Diffusion Cell.

4. The in vitro method of Claim 1, 2 or 3, wherein the first skin explant comprises an epidermal layer having an outer stratum corneum and an upper dermis, and wherein the surface is the stratum corneum.

5. The in vitro method of Claim 1, 2, 3 or 4, wherein the second skin explant comprises an epidermal layer having an outer stratum corneum, an upper dermis, a lower dermis and a subcutaneous layer, and wherein the surface is the stratum corneum.

6. The in vitro method of Claim 5, wherein step (i) comprises determining the concentration of the compound within the upper dermis of the first skin explant using a static Franz Diffusion Cell.

7. The in vitro method of Claim 6, wherein step (ii) comprises determining the concentration of the compound within the lower dermis and/or subcutaneous layer of the second skin explant using open flow micro-perfusion (OFM).

8. The in vitro method of Claim 7, wherein the step of determining the concentration of the compound within the first and second skin explants is repeated a plurality of times over a time period of at least 12, preferable at least 24 hours.

9. The in vitro method of any of the preceding claims, further comprising the step of generating a time-resolved profile of the concentrations of the compound.

10. The in vitro method of any preceding claim, wherein the compound is part of a topical composition comprising at least one pharmaceutically acceptable carrier, excipient or diluent that is applied to the surface of the first and second skin explants.

11. The in vitro method of any of the preceding claims, wherein the first and second skin explants are human skin explants.

12. An in vitro method for comparing the effect of excipients on diffusion of a compound into the skin, comprising:

(i) applying a first formulation comprising the compound to the stratum corneum of a skin explant, determining the concentration of the compound within the upper dermis of the skin explant at different time-points over a period of time using a Diffusion Cell (DC);
(ii) applying the first formulation comprising the compound to the stratum corneum of a skin explant, determining the concentration of the compound within the lower dermis and subcutis of the skin explant at different time-points over a period of time using open flow micro-perfusion (OFM);
(iii) applying a second formulation comprising the compound to the stratum corneum of a skin explant, determining the concentration of the compound within the upper dermis of the skin explant at different time-points over a period of time using a Diffusion Cell (DC);
(iv) applying the second formulation comprising the compound to the stratum corneum of a skin explant, determining the concentration of the compound within the lower dermis and subcutis of the skin explant at different time-points over a period of time using open flow micro-perfusion (OFM);
(v) combining the concentrations determined in (i) and (ii) to generate a first time-resolved profile of the concentration of the compound as a function of time within the upper dermis, lower dermis and subcutis of the skin explants;
(vi) combining the concentrations determined in (ii) and (iii) to generate a second time-resolved profile of the concentration of the compound as a function of time within the upper dermis, lower dermis and subcutis of the skin explants; and
(vii) comparing the first and second time-resolved profiles;

wherein steps (i), (ii), (iii) and (iv) are performed in any order (sequentially or in parallel), step (v) is performed after both steps (i) and (ii) are completed, step (vi) is performed after both steps (iii) and (iv) are completed and wherein step (vii) is performed after steps (v) and (vi) are completed.

13. The in vitro method of Claim 12 for use in determining bioequivalence, wherein the compound is an active pharmaceutical ingredient (API), and wherein one of the first or second formulations is a topical formulation comprising the API and the other of the first or second formulations is a test formulation comprising the API.

14. An in vitro method for determining a skin permeation profile for a topical pharmaceutical composition comprising an active ingredient and at least one pharmaceutically acceptable carrier, the method comprising:

(i) obtaining time-resolved concentration data for the active ingredient within the upper dermis of a first skin explant, wherein the concentration data is obtained using a diffusion cell;
(ii) obtaining time-resolved concentration data for the active ingredient within the lower dermis and/or subcutis of a second skin explant, wherein the concentration data is obtained using open flow micro-perfusion;
(iii) generating a time-resolved profile of concentration data for the active ingredient as a function of time within the upper dermis, lower dermis and/or subcutis.

15. The in vitro method of any one of claims 1 to 14, wherein the active pharmaceutical ingredient is diclofenac or a salt thereof, particularly diclofenac sodium or diclofenac diethylammonium.

*Fig. 1*

*Fig. 2*

*Fig. 3*

*Fig. 4*

*Fig. 5*

*Fig. 6*

Fig. 7(a)

Fig. 7(b)

EP 4 246 142 A1

Formulation F

FDC: 0.3 ± 0.1 mm
OFM dermis: 1.12 ± 0.12 mm
OFM subcutis: 3.21 ± 0.45 mm

*Fig. 7(c)*

*Fig. 8*

Fig. 9

Fig. 10

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | HUMMER JOANNA ET AL: "Optimization of topical formulations using a combination of in vitro methods to quantify the transdermal passive diffusion of drugs", INTERNATIONAL JOURNAL OF PHARMACEUTICS, ELSEVIER, NL, vol. 620, 10 April 2022 (2022-04-10), XP087052016, ISSN: 0378-5173, DOI: 10.1016/J.IJPHARM.2022.121737 [retrieved on 2022-04-10] * the whole document * | 1-15 | INV. G01N33/50 |
| A | KOPECNÁ MONIKA ET AL: "Transdermal Permeation and Skin Retention of Diclofenac and Etofenamate/Flufenamic Acid From Over-the-Counter Pain Relief Products", JOURNAL OF PHARMACEUTICAL SCIENCES, vol. 110, no. 6, 1 June 2021 (2021-06-01), pages 2517-2523, XP055956988, US ISSN: 0022-3549, DOI: 10.1016/j.xphs.2021.01.022 Retrieved from the Internet: URL:http://dx.doi.org/10.1016/j.xphs.2021.01.022> * page 2518 – page 2522; figures * | 1-15 | |
| | | | **TECHNICAL FIELDS SEARCHED (IPC)** |
| | | | G01N |

-/--

The present search report has been drawn up for all claims

| Place of search | Date of completion of the search | Examiner |
|---|---|---|
| Munich | 5 September 2022 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 22 16 1997

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOLMGAARD R ET AL: "Comparison of Open-Flow Microperfusion and Microdialysis Methodologies When Sampling Topically Applied Fentanyl and Benzoic Acid in Human Dermis", PHARMACEUTICAL RESEARCH, KLUWER ACADEMIC PUBLISHERS-PLENUM PUBLISHERS, NL, vol. 29, no. 7, 15 March 2012 (2012-03-15) , pages 1808-1820, XP035067731, ISSN: 1573-904X, DOI: 10.1007/S11095-012-0705-9 * page 1808 - page 1817; figures * | 1-15 | |
| X | Patel Tulsi Rashesh: "A Review of Bioequivalence Assessment Techniques for Topical Drug Products", University of Georgia?ProQuest Dissertations Publishing, ?2020.?28154781, 1 January 2020 (2020-01-01), pages 1-54, XP055956999, ISBN: 979-8-5699-1759-4 Retrieved from the Internet: URL:XP055956999 [retrieved on 2022-09-01] * page 10 - page 19; figures 2 ,3, 4, 9 * * page 31 - page 35 * | 1-15 | |
| X | SCHWINGENSCHUH SIMON ET AL: "Assessment of skin permeability to topically applied drugs by skin impedance and admittance", PHYSIOLOGICAL MEASUREMENT, vol. 38, 1 November 2017 (2017-11-01), pages N138-N150, XP055957295, DOI: 10.1088/1361-6579/aa904e Retrieved from the Internet: URL:https://iopscience.iop.org/article/10. 1088/1361-6579/aa904e/pdf> * page N139 - page N141; figures * | 1-15 | |

TECHNICAL FIELDS SEARCHED (IPC)

The present search report has been drawn up for all claims

| Place of search | Date of completion of the search | Examiner |
|---|---|---|
| Munich | 5 September 2022 | Gonçalves Mauger, M |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

...............................................................................

& : member of the same patent family, corresponding
   document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007131780 A
- US 5547351 A
- US 5198109 A